# EUROPÄISCHE PATENTSCHRIFT

(11) **EP 0 480 237 B1**
(45) Veröffentlichungstag und Bekanntmachung des Hinweises auf die Patenterteilung: **30.11.1994**
(21) Anmeldenummer: 91116250.1
(22) Anmeldetag: 24.09.1991
(51) Int. Cl.: C07C 39/17, C08G 64/06

(54) **Spezielle Dihydroxydiphenylbicycloalkane, ihre Herstellung und ihre Verwendung zur Herstellung von hochmolekularen Polycarbonaten**
Particular dihydroxydiphenylbicycloalkanes, their preparation and their use for the preparation of high molecular polycarbonates
Dihydroxydiphénylbicycloalcanes particuliers, leur préparation et leur utilisation pour la fabrication de polycarbonates à haut poids moléculaire

(30) Priorität: 06.10.1990 DE 4031756
(43) Veröffentlichungstag der Anmeldung: 15.04.1992
(73) Patentinhaber: BAYER AG, 51368 Leverkusen (DE)
(72) Erfinder: Serini, Volker, Dr., W-4150 Krefeld (DE); Westeppe, Uwe, Dr., W-4040 Mettmann (DE); Fengler, Gerd, Dr., W-4150 Krefeld-Traar (DE); Hajek, Manfred, Dr., W-5090 Leverkusen (DE); Casser, Carl, Dr., W-5000 Köln 80 (DE); Waldmann, Helmut, Dr., W-5090 Leverkusen (DE)

(56) Entgegenhaltungen:
- EP-A- 0 180 133
- EP-A- 0 359 953
- DE-A- 3 035 204
- DE-A- 3 343 898
- DE-A- 3 344 326
- DE-B- 2 926 593
- PATENT ABSTRACTS OF JAPAN, unexamined applications, C field, Band 13, Nr. 419, 18. September 1989 THE PATENT OFFICE JAPANESE GOVERNMENT Seite 91 C 637

## Beschreibung

Gegenstand der vorliegenden Erfindung sind Dihydroxydiphenylbicycloalkane der Formel (I) 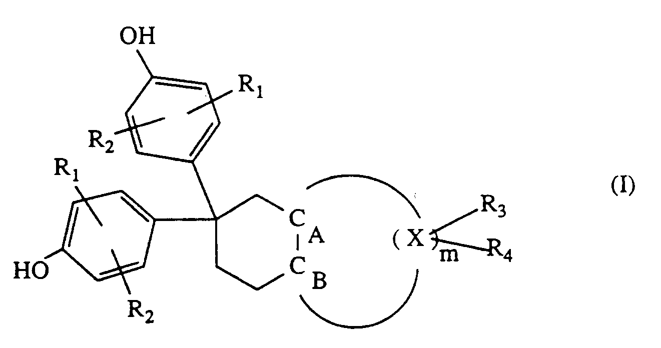
worin
- R₁ und R₂: unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl, und C₇-C₁₂-Aralkyl, bevorzugt Phenyl-C₁-C₄-Alkyl, insbesondere Benzyl,
- m: eine ganze Zahl von 3 bis 7, bevorzugt 3 oder 4,
- R₃ und R₄: für jedes X individuell wählbar, unabhängig voneinander Wasserstoff, lineares oder verzweigtes C₁-C₆-Alkyl, C₆-C₁₀-Aryl, bevorzugt Phenyl, oder Aralkyl
und
- X: Kohlenstoff bedeuten,
mit der Maßgabe, daß an mindestens einem Atom X die Substituenten R₃ und R₄ nicht gleichzeitig Wasserstoff bedeuten.

Bevorzugt ist an 1 bis 2 Atomen X, insbesondere an zwei Atomen X, R₃ und R₄ gleichzeitig Alkyl. Bevorzugter Alkylrest ist Methyl. Die X-Atome in α-Stellung zu den beiden Brücken-C-Atomen (C_{A} und C_{B}) sind bevorzugt dialkylsubsituiert. Besonders bevorzugt ist, daß ein X-Atom in α-Stellung zu C_{A} oder C_{B} mono- oder dialkylsubstituiert ist.

Insbesondere sind Gegenstand der Erfindung Dihydroxydiphenylbicycloalkane mit 9 und 10 Ring-C-Atomen im bicycloaliphatischen Rest (m = 3 oder 4 in Formel (I)) wie beispielsweise die Diphenole der Formeln 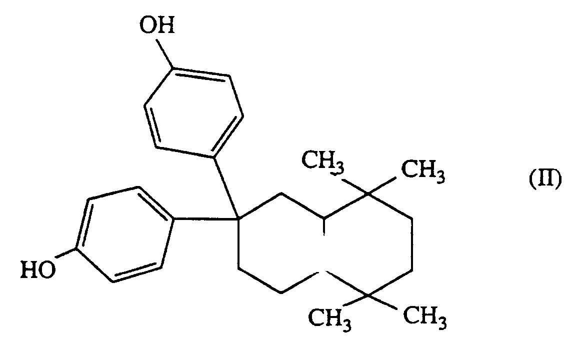 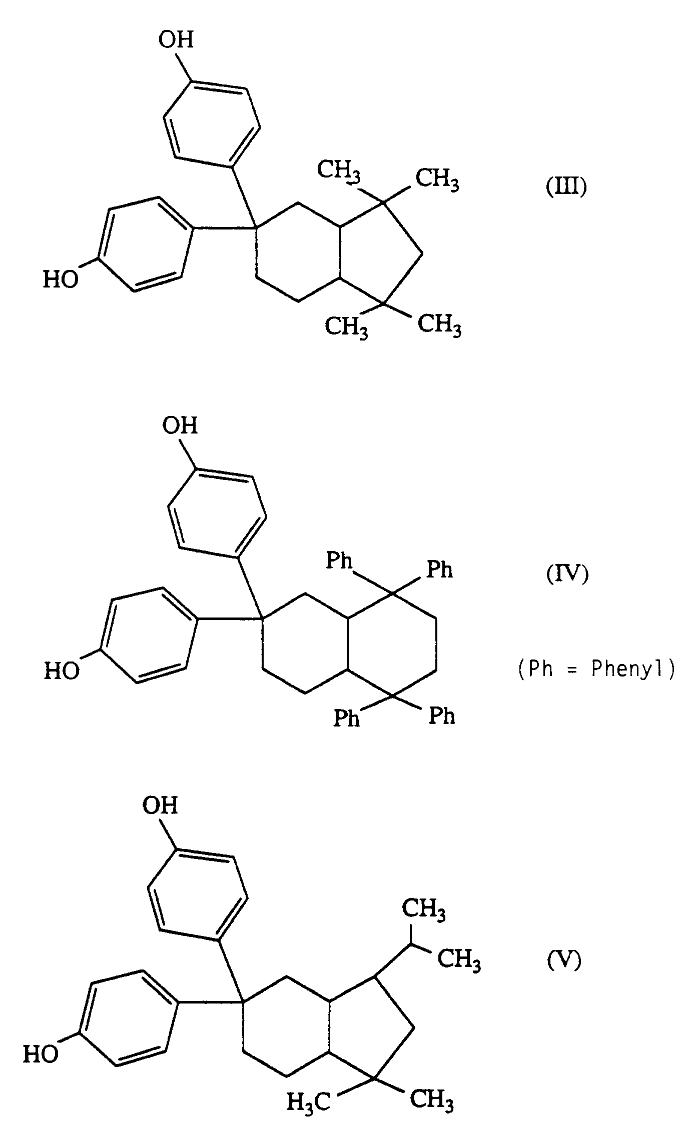 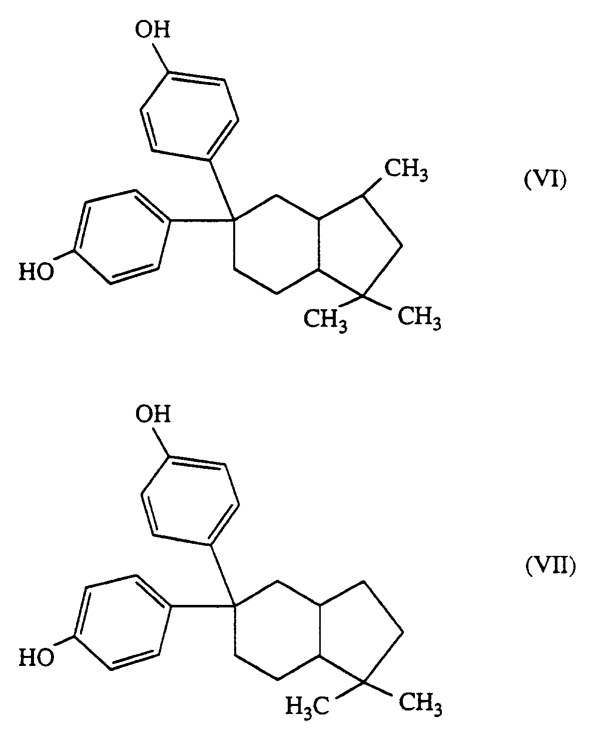
Die erfindungsgemäßen Dihydroxydiphenylbicycloalkane der Formel (I) können in an sich bekannter Weise durch Kondensation von Phenolen der Formel (VIII) 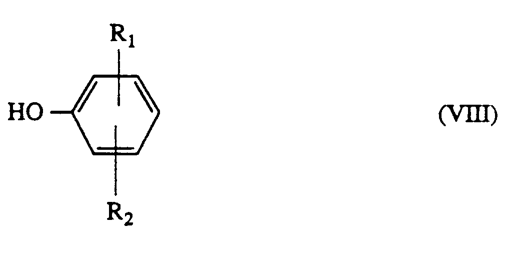
und Ketonen der Formel (IX) 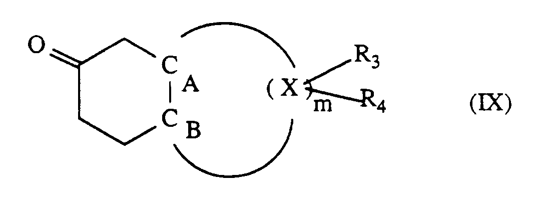
hergestellt werden, wobei in den Formeln (VII) und (IX) X, R₁, R₂, R₃, R₄ und m die für Formel (I) angegebene Bedeutung haben.

Die Phenole der Formel (VIII) sind entweder literaturbekannt oder nach literaturbekannten Verfahren erhältlich (siehe beispielsweise für Kresole und Xylenole, Ullmanns Enzyklopädie der technischen Chemie 4. neubearbeitete und erweiterte Auflage Band 15, Seiten 61-77, Verlag Chemie-Weinheim-New York 1978; für Chlorphenole, Ullmanns Enzylklopädie der technischen Chemie, 4. Auflage, Verlag Chemie, 1975, Band 9, Seiten 573-582; und für Alkylphenole, Ullmanns Enzyklopädie der technischen Chemie, 4. Auflage, Verlag Chemie 1979, Band 18, Seiten 191-214).

Beispiele für geeignete Phenole der Formel (VIII) sind:
Phenol, o-Kresol, m-Kresol, 2,6-Dimethylphenol, 2-Chlorphenol, 3-Chlorphenol, 2,6-Dichlorphenol, 2-Cyclohexylphenol, Diphenylphenol und o-Benzylphenol.

Die Ketone der Formel (IX) lassen sich aus den literaturbekannten (siehe z.B. Houben-Weyl, Methoden der organischen Chemie, Band VI/1c, G. Thieme Verlag Stuttgart 1976, Seite 925-1022) Phenolen der Formel (X), 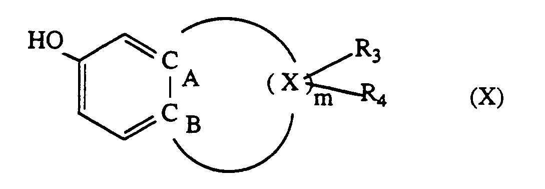
worin R₃, R₄, X und m die obige Bedeutung haben, nach der allgemein bekannten Reaktionsfolge-Hydrierung des aromatischen Ringes zum sekundären Alkohol und anschließende Oxidation zum Keton darstellen. Eine direkte Hydrierung der Phenole der Formel (X) zu den Ketonen der Formel (IX) ist ebenfalls möglich (siehe hierzu: Houben-Weyl, Methoden der organischen Chemie, vierte Auflage Band 4/1c Seite 177-188, G.Thieme Verlag Stuttgart 1980).

Beispiele für geeignete Ketone der Formel (IX) sind: 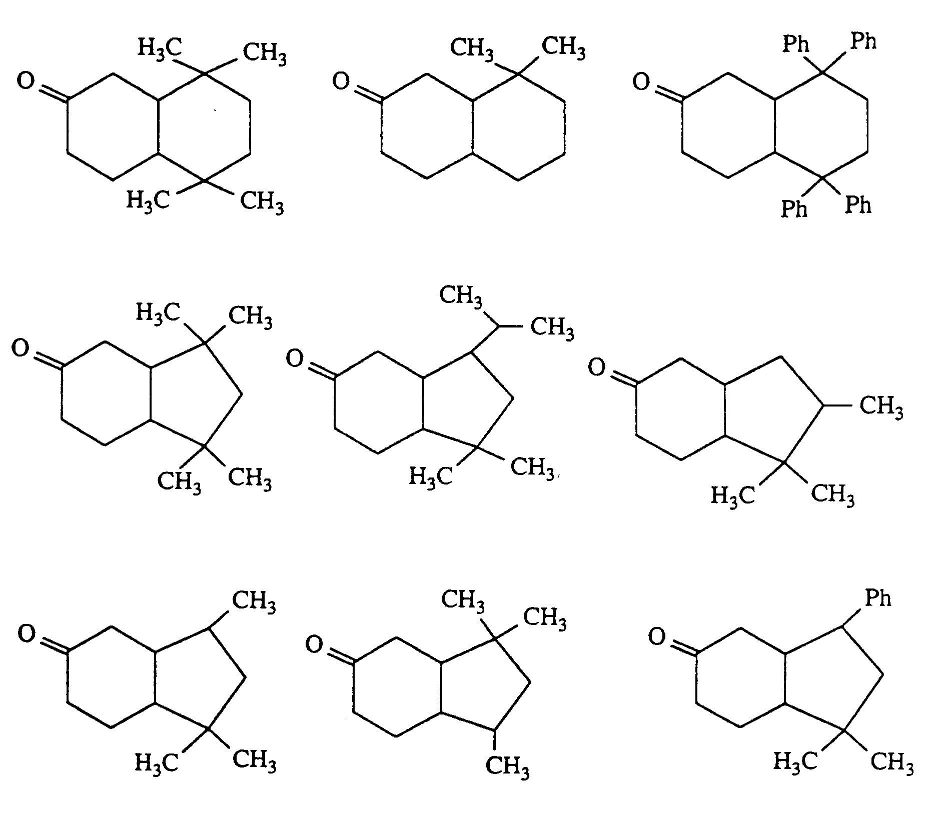
Zur Bisphenolherstellung werden im allgemeinen 2 bis 30 Mol vorzugsweise 2,5 bis 20 Mol Phenol (VIII) pro Mol Keton (IX), verwendet Bevorzugte Reaktionszeiten betragen 1 bis 300 Stunden. Im allgemeinen arbeitet man bei Temperaturen von -30°C bis 300°C, vorzugsweise von -15°C bis 150°C und bei Drücken von 1 bis 20 bar, vorzugsweise von 1 bis 10 bar.

Die Kondensation wird im allgemeinen in Gegenwart saurer Katalysatoren durchgeführt. Beispiele sind Chlorwasserstoff, Bromwasserstoff, Fluorwasserstoff, Bortrifluorid, Aluminiumtrichlorid, Zinkdichlorid, Titantetrachlorid, Zinntetrachlorid, Phosphorhalogenide, Phosphorpentoxid, Phosphorsäure, konzentrierte Salzsäure oder Schwefelsäure, aromatische oder aliphatische Sulfonsäuren, Mischungen aus Essigsäure und Acetanhydrid sowie aromatischen oder aliphatischen Sulfonsäuren. Die Verwendung saurer Ionenaustauscher, saurer Zeolithe oder Trimethylchlorsilan ist ebenfalls möglich.

Weiterhin kann die Umsetzung durch Zugabe von Co-Katalysatoren wie C₁-C₁₈-Alkyl-Mercaptanen, Schwefelwasserstoff, Thiophenolen, Thiosäuren und Dialkylsulfiden in Mengen von 0,001 bis 0,4 Mol/Mol Keton, insbesondere von 0,01 bis 0,2 Mol/Mol Keton beschleunigt werden.

In den Fällen, in denen der Katalysator gleichzeitig als wasserentziehendes Mittel fungiert, ist es nicht erforderlich, zusätzlich wasserentziehende Mittel einzusetzen, letzteres ist jedoch zur Erzielung guter Umsätze in jedem Fall dann vorteilhaft, wenn der eingesetzte Katalysator das Reaktionswasser nicht bindet.

Geeignete wasserentziehende Mittel sind beispielsweise Acetanhydrid, Zeolithe, Polyphosphorsäure und Phosphorpentoxid.

Die Kondensation kann ohne Lösungsmittel oder in Gegenwart eines inerten Lösungsmittels (z.B. aliphatischer und aromatischer Kohlenwasserstoff, Chlorkohlenwasserstoff) durchgeführt werden.

In manchen Fällen verläuft die Reaktion nicht ganz einheitlich, d.h. es können mehrere, unterschiedliche Produkte entstehen, so daß die gewünschte Verbindung zunächst aus einem Gemisch isoliert werden muß. Für Einzelheiten der Kondensation sei auf Schnell Chemistry and Physics of Polycarbonates, Intescience Publishers, New York 1964, verwiesen. Manchmal kann die Reaktion durch Wahl entsprechender Katalysatoren und Reaktionsbedingungen so gesteuert werden, daß die gewünschte Verbindung ausfällt oder auskristallisiert, was deren Isoleirung erleichtert.

Gegenstand der vorliegenden Erfindung ist somit außerdem ein Verfahren zur Herstellung der Dihydroxydiphenylbicycloalkane der Formel (I) 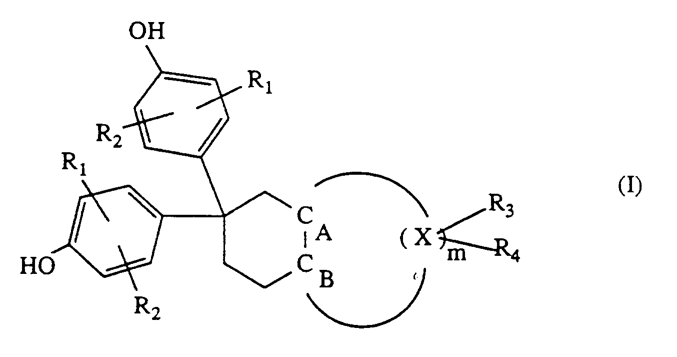
worin
- R₁ und R₂: unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl, und C₇-C₁₂-Aralkyl, bevorzugt Phenyl-C₁-C₄-Alkyl, insbesondere Benzyl,
- m: eine ganze Zahl von 3 bis 7, bevorzugt 3 oder 4.
- R₃ und R₄: für jedes X individuell wählbar, unabhängig voneinander Wasserstoff, lineares oder verzweigtes C₁-C₆-Alkyl, C₆-C₁₀-Aryl, bevorzugt Phenyl, oder Aralkyl
und
- X: Kohlenstoff bedeuten,
mit der Maßgabe, daß an mindestens einem Atom X die Substituenten R₃ und R₄ nicht gleichzeitig Wasserstoff bedeuten,
das dadurch gekennzeichnet ist, daß man Phenole der Formel (VIII) 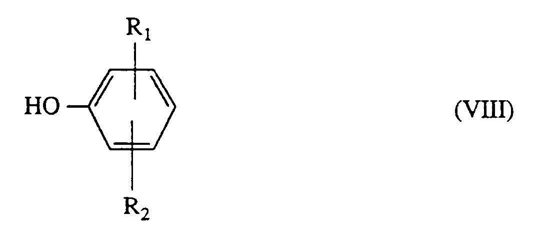
mit Ketonen der Formel (IX) 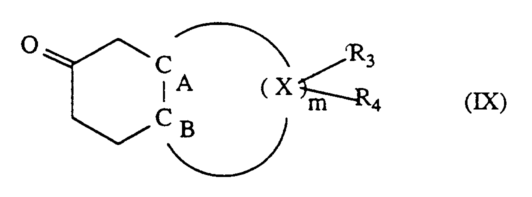
worin X, m, R₁, R₂, R₃ und R₄ die für Formel (I) angegebene Bedeutung haben, im Molverhältnis (VIII):(IX) von 2:1 bis 30:1, vorzugsweise zwischen 2,5:1 und 20:1 bei Temperaturen zwischen -30°C und 300°C, vorzugsweise zwischen -15°C und 150°C und bei Drücken von 1 bis 20 bar, vorzugsweise von 1 bis 10 bar in Anwesenheit saurer Katalysatoren und gegebenenfalls in Anwesenheit von Co-Katalysatoren und/oder Lösungsmitteln und/oder wasserentziehenden Mitteln umsetzt.

Bevorzugt ist an 1 bis 2 Atomen X, insbesondere an zwei Atomen X, R₃ und R₄ gleichzeitig Alkyl. Bevorzugter Alkylrest ist Methyl. Die X-Atome in α-Stellung zu den beiden Brücken-C-Atomen (C_{A} und C_{B}) sind bevorzugt dialkylsubstituiert. Besonders bevorzugt ist, daß ein X-Atom in α-Stellung zu C_{A} oder C_{B} mono- oder dialkylsubstituiert ist.

### Herstellung der Dihydroxydiphenylbicycloalkane

Die Struktur der erfindungsgemäßen Dihydroxydiphenylbicycloalkane steht im Einklang mit den Ergebnissen der ¹H-NMR- und massenspektroskopischen Untersuchunen.

### Beispiel A1

### Herstellung des Diphenols 1,1,4,4-Tetramethyl-7,7-bis-(4-hydroxyphenyl)-decalin (II)

In einer Rührapparatur mit Rührer, Thermometer, Rückflußkühler werden 282 g (3 Mol) Phenol, 104 g (0,5 Mol) 1,1,4,4-Tetramethyl decalin-7-on und 10,1 g (0,05 Mol) Dodecylthiol bei 26°C vorgelegt Zu dieser Lösung werden bei 30 g 37 %ige HCl gegeben. Das Reaktionsgemisch wird bei 20 bis 35°C bis zu 95 %igem Umsatz zugerührt Das Reaktionsgemisch wird mehrfach mit Wasser extrahiert. Das resultierende Phenol-Bisphenol-Addukt wird durch mehrmaliges Waschen mit Hexan von Phenol und Nebenprodukten weitgehend befreit und anschließend aus Toluol umkristallisiert.
Ausbeute: 153 g
Schmelzpunkt: 188 bis 191°C.

### Beispiel 2

### Herstellung des Diphenols 1,1,4,4-Tetramethyl-7,7-bis-(3,5-dimethyl-4-hydroxyphenyl)-decalin

Analog zu Beispiel 1 werden anstelle von 3 Mol Phenol 3 Mol 2,6-Dimethylphenol eingesetzt, wobei die Umsetzung bei 40°C durchgeführt wurde.

Schmelzpunkt: 229 bis 234°C.

### Beispiel 3

### Herstellung des Diphenols 1,1,3,3-Tetramethyl-5,5-bis-(4-hydroxyphenyl)-indan (III)

Analog zu Beispiel 1 werden anstelle von 0,5 Mol 1,1,4,4-Tetramethyldecalin-7-on 0,5 Mol 1,1,3,3-Tetramethylindan-5-on eingesetzt.

Schmelzpunkt: 208 bis 209°C.

Die erfindungsgemäßen Diphenole der Formel (I) sind insbesondere geeignet zur Herstellung von hochmolekularen, thermoplstischen Polycarbonaten, die sich durch ein gutes Eigenschaftsbild auszeichnen.

Gegenstand der vorliegenden Erfindung ist somit auch die Verwendung der Diphenole der Formel (I) zur Herstellung von hochmolekularen thermoplastischen, aromatischen Polycarbonaten.

Es können sowohl ein Diphenol der Formel (I) unter Bildung von Homopolycarbonaten als auch mehrere Diphenole der Formel (I) unter Bildung von Copolycarbonaten verwendet werden.

Außerdem können die Diphenole der Formel (I) auch im Gemisch mit anderen Diphenolen, beispielsweise mit denen der Formel HO-Z-OH (IX), zur Herstellung von hochmolekularen, thermoplastischen, aromatischen Polycarbonaten verwendet werden.

Geeignete andere Diphenole der Formel HO-Z-OH (XI) sind solche, in denen Z ein aromatischer Rest mit 6 bis 30 C-Atomen ist, der einen oder mehrere aromatischen Kerne enthalten kann, substituiert sein kann und aliphatische Reste oder andere cycloaliphatische Reste als die der Formel (I) oder Heteroatome als Brückenglieder enthalten kann.

Beispiele für Diphenole der Formel (XI) sind
Hydrochinon,
Resorcin,
Dihydroxydiphenyle
Bis-(hydroxyphenyl)-alkane,
Bis-(hydroxyphenyl)-cycloalkane,
Bis-(hydroxyphenyl)-sulfide,
Bis-(hydroxyphenyl)-ether,
Bis-(hydroxyphenyl)-ketone,
Bis-(hydroxyphenyl)-sulfone,
Bis-(hydroxyphenyl)-sulfoxide,
α,α'-Bis-(hydroxyphenyl)-diisopropylbenzole
sowie deren kernalkylierte und kernhalogenierte Verbindungen.

Diese und weitere geeignete andere Diphenole sind z.B. in den US-PS 3 028 365, 2 999 835, 3 148 172, 3 275 601, 2 991 273, 3 271 367, 3 062 781, 2 970 131 und 2 999 846, in den deutschen Offenlegungsschriften 1 570 703, 2 063 050, 2 063 052, 2 211 056, 3 832 396, der französischen Patentschrift 1 561 518 und in der Monographie H. Schnell, Chemistry and Physics of Polycarbonates, Interscience Publishers, New York 1964", beschrieben.

Bevorzugte andere Diphenole sind beispielsweise:
4,4',-Dihydroxydiphenyl,
2,2-Bis-(4-hydroxyphenyl)-propan,
2,4-Bis-(4-hydroxyphenyl)-2-methylbutan,
1,1-Bis-(4-hydroxyphenyl)-cyclohexan,
α,α'-Bis-(4-hydroxyphenyl)-p-diisopropylbenzol,
2,2-Bis-(3-methy1-4-hydroxyphenyl)-propan,
2,2-Bis-(3-chlor-4-hydroxyphenyl)-propan,
Bis-(3,5-dimethyl-4-hydroxyphenyl)-methan,
2,2-Bis-(3,5-dimethyl-4-hydroxyphenyl)-propan,
Bis-(3,5-dimethyl-4-hydroxyphenyl)-sulfon,
2,4-Bis-(3,5-dimethyl-4-hydroxyphenyl)-2-methylbutan,
1,1-Bis-(3,5-dimethyl-4-hydroxyphenyl)-cyclohexan,
1,1-Bis-(4-hydroxyphenyl)-3,3,5-trimethylcyclohexan,
α,α'-Bis(3,5-dimethyl-4-hydroxyphenyl)-p-diisopropylbenzol,
2,2-Bis-(3,5-dichlor-4-hydroxyphenyl)-propan und
2,2-Bis-(3,5-dibrom-4-hydroxyphenyl)-propan.

Besonders bevorzugte Diphenole der Formel (XI) sind beispielsweise:
2,2-Bis-(4-hydroxyphenyl)-propan,
2,2-Bis-(3,5-dimethyl-4-hydroxyphenyl)-propan,
2,2-Bis-(3,5-dichlor-4-hydroxyphenyl)-propan,
2,2-Bis-(3,5-dibrom-4-hydroxyphenyl)-propan,
1,1-Bis-(4-hydroxyphenyl)-cyclohexan.

Insbesondere ist 2,2-Bis-(4-hydroxyphenyl)-propan bevorzugt.

Die anderen Diphenole können sowohl einzeln als auch im Gemisch eingesetzt werden.

Das molare Verhältnis von erfindungsgemäß zu verwendenden Diphenolen der Formel (I) zu den gegebenenfalls mitzuverwendenden anderen Diphenolen, beispielsweise denen der Formel (XI), soll zwischen 100 Mol-% (I) zu 0 Mol-% anderem Diphenol und 2 Mol-% (I) zu 98 Mol-% anderem Diphenol, vorzugsweise zwischen 100 Mol-% (I) zu 0 Mol-% anderem Diphenol und 5 Mol-% (I) zu 95 Mol-% anderem Diphenol und insbesondere zwischen 100 Mol-% (I) zu 0 Mol-% anderem Diphenol und 10 Mol-% (I) zu 90 Mol-% anderem Diphenol und ganz besonders zwischen 100 Mol-% (I) zu 0 Mol-% anderem Diphenol und 20 Mol-% (I) zu 80 Mol-% anderem Diphenol liegen.

Die hochmolekularen Polycarbonate aus den erfindungsgemäßen Diphenolen der Formel (I), gegebenenfalls in Kombination mit anderen Diphenolen können nach den bekannten Polycarbonatherstellungsverfahren hergestellt werden. Dabei können die verschiedenen Diphenole sowohl statistisch als auch blockweise miteinander verknüpft sein.

Gegenstand der vorliegenden Erfindung ist somit auch ein Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, vorzugsweise nach dem Zweiphasengrenzflächenverfahren, das dadurch gekennzeichnet ist, daß man als Diphenole solche der Formel (I) in Mengen von
100 Mol-% bis 2 Mol-%, vorzugsweise in Mengen von
100 Mol-% bis 5 Mol-% und insbesondere in Mengen von
100 Mol-% bis 10 Mol-% und ganz besonders
100 Mol-% bis 20 Mol-%, bezogen jeweils auf die Gesamtmolmenge an eingesetzten Diphenolen, verwendet.

Als Kettenabbrecher zur Regelung des Molekulargewichts dienen in bekannter Weise monofunktionelle Verbindungen in üblichen Konzentrationen. Geeignete Verbindungen sind z.B. Phenol, tert.-Butylphenole oder andere Alkyl-C₁-C₇-substituierte Phenole.

Zur Regelung des Molekulargewichts sind insbesondere kleine Mengen Phenole der Formel (XII) geeignet 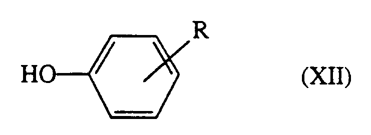
worin R einen verzweigten C₈- und/oder C₉-Alkylrest darstellt. Bevorzugt ist im Alkylrest R der Anteil an CH₃-Protonen zwischen 47 und 89 % und der Anteil der CH- und CH₂-Protonen zwischen 53 und 11 %; ebenfalls bevorzugt ist R in o- und/oder p-Stellung zu OH-Gruppe, und besonders bevorzugt die obere Grenze des ortho-Anteils 20 %. Die Kettenabbrecher werden im allgemeinen in Mengen von 0,5 bis 10, bevorzugt 1,5 bis 8 Mol-%, bezogen auf eingesetzte Diphenole, eingesetzt.

Als Verzweiger dienen, falls benutzt, in bekannter Weise geringe Mengen, vorzugsweise Mengen zwischen 0,05 und 2,0 Mol-% (bezogen auf eingesetzte Diphenole), an drei oder mehr als dreifunktionellen Verbindungen, insbesondere solchen mit drei oder mehr als drei phenolischen Hydroxylgruppen, um verzweigte Polycarbonate zu erhalten. Einige der verwendbaren Verbindungen mit drei oder mehr als drei phenolischen Hydroxylgruppen sind
Phloroglucin,
4,6-Dimethyl-2,4,6-tri-(4-hydroxyphenyl)-hepten-2,
4,6-Dimethyl-2,4,6-tri-(4-hydroxyphenyl)-heptan,
1,3,5-Tri-(4-hydroxyphenyl)-benzol,
1,1,1-Tri-(4-hydroxyphenyl)-ethan,
Tri-(4-hydroxyphenyl)-phenylmethan,
2,2,-Bis-(4,4-bis-(4-hydroxyphenyl)-cyclohexyl)-propan,
2,4-Bis-(4-hydroxyphenyl-isopropyl)-phenol,
2,6-Bis-(2-hydroxy-5'-methyl-benzyl)-4-methylphenol,
2-(4-Hydroxyphenyl)-2-(2,4-dihydroxyphenyl)-propan,
Hexa-(4-(4-hydroxyphenyl-isopropyl)-phenyl)-orthoterephthal-säureester,
Tetra-(4-hydroxyphenyl)-methan,
Tetra-(4-(4-hydroxyphenyl-isopropyl)-phenoxy)-methan und
1,4-Bis-((4'-,4''-dihydroxytriphenyl)-methyl)-benzol.

Einige der sonstigen dreifunktionellen Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Die erfindungsgemäßen Polycarbonate können vorzugsweise nach dem Phasengrenzflächenverfahren (vgl. H. Schnell, "Chemistry and Physics of Polycarbonates", Polymer Reviews, Vol. IX, Seite 33 ff., Interscience Publ., 1964) in an sich bekannter Weise hergestellt werden. Hierbei werden die Diphenole der Formel (I) in wäßrig alkalischer Phase gelöst. Zur Herstellung von Co-Polycarbonaten mit anderen Diphenolen werden Gemische von Diphenolen der Formel (I) und den anderen Diphenolen, beispielsweise denen der Formel (XI), eingesetzt. Zur Regulierung des Molekulargewichtes können Kettenabbrecher z.B. der Formel (XII) zugegeben werden. Dann wird in Gegenwart einer inerten, vorzugsweise Polycarbonat lösenden, organischen Phase mit Phosgen nach der Methode der Phasengrenzflächenkondensation umgesetzt. Die Reaktionstemperatur liegt zwischen 0°C und 40°C.

Die gegebenenfalls mitzuverwendenden 0,05 bis 2 Mol-% an Verzweigern können entweder mit den Diphenolen in der wäßrig alkalischen Phase vorgelegt werden oder in dem organischen Lösungsmittel gelöst vor der Phosgenierung zugegeben werden.

Neben den einzusetzenden Diphenolen der Formel (I) sowie den anderen Diphenolen (XI) können auch deren Mono- und/oder Bis-chlorkohlensäureester mitverwendet werden, wobei diese in organischen Lösungsmitteln gelöst zugegeben werden. Die Menge an Kettenabbrechern sowie an Verzweigern richtet sich dann nach Molen Diphenolat-Struktureinheiten von (I) und gegebenenfalls von den anderen Diphenolen, wie beispielsweise von (XI); ebenso kann bei Einsatz von Chlorkohlensäureestern die Phosgenmenge in bekannter Weise entsprechend reduziert werden.

Geeignete organische Lösungsmittel für die Lösung der Kettenabbrecher sowie gegebenenfalls für die Verzweiger und die Chlorkohlensäureester sind beispielsweise Methylenchlorid, Chlorbenzol, Aceton, Acetonitril sowie Mischungen dieser Losungsmittel, insbesondere Mischungen aus Methylenchlorid und Chlorbenzol. Gegebenenfalls können die verwendeten Kettenabbrecher und Verzweiger im gleichen Solvens gelöst werden.

Als organische Phase für die Phasengrenzflächenpolykondensation dient beispielsweise Methylenchlorid, Chlorbenzol sowie Mischungen aus Methylenchlorid und Chlorbenzol.

Als wäßrige alkalische Phase dient beispielsweise wäßrige NaOH-Lösung.

Die Herstellung der erfindungsgemäßen Polycarbonate nach dem Phasengrenzflächenverfahren kann in üblicher Weise durch Katalysatoren wie tertiäre Amine, insbesondere tertiäre aliphatische Amine wie Tributylamin oder Triethylamin katalysiert werden; die Katalysatoren können in Mengen von 0,05 bis 10 Mol-%, bezogen auf Mole an eingesetzten Diphenolen eingesetzt werden. Die Katalysatoren können vor Beginn der Phosgenierung oder während oder auch nach der Phosgenierung zugesetzt werden.

Die Isolierung der erfindungsgemäßen Polycarbonate erfolgt in bekannter Weise.

Die erfindungsgemäßen hochmolekularen, thermoplastischen, aromatischen Polycarbonate können auch nach dem bekannten Verfahren in homogener Phase, dem sogenannten "Pyridinverfahren" sowie nach dem bekannten Schmelzumesterungsverfahren unter Verwendung von beispielsweise Diphenylcarbonat anstelle von Phosgen hergestellt werden. Auch hier werden die erfindungsgemäßen Polycarbonate in bekannter Weise isoliert.

Die nach dem erfindungsgemäßen Verfahren erhältlichen Polycarbonate haben bevorzugt Molekulargewichte M_{w} (Gewichtsmittel, ermittelt durch Gelchromatographie nach vorheriger Eichung) von mindestens 9.000, besonders bevorzugt von 12.000 bis 190.000 und insbesondere von 19.000 bis 65.000. Sie können linear oder verzweigt sein, sie sind Homopolycarbonate oder Copolycarbonate auf Basis der Diphenole der Formel (I).

Gegenstand der vorliegenden Erfindung sind somit auch hochmolekulare, thermoplastische, aromatische Polycarbonate mit M_{w} (Gewichtsmittelmolekulargewichten) von mindestens 9.000, vorzugsweise von 12.000 bis 190.000 und insbesondere von 19.000 bis 65.000, erhältlich nach dem erfindungsgemäßen Verfahren aus Diphenolen der Formel (I), die linear oder verzweigt sind.

Gegenstand der vorliegenden Erfindung sind somit auch hochmolekulare, thermoplastische, aromatische Polycarbonate mit M_{w} (Gewichtsmittelmolekulargewichten) von mindestens 9.000, vorzugsweise von 12.000 bis 190.000 und insbesondere von 19.000 bis 65.000, die bifunktionelle Carbonatstruktureinheiten der Formel (Ia) 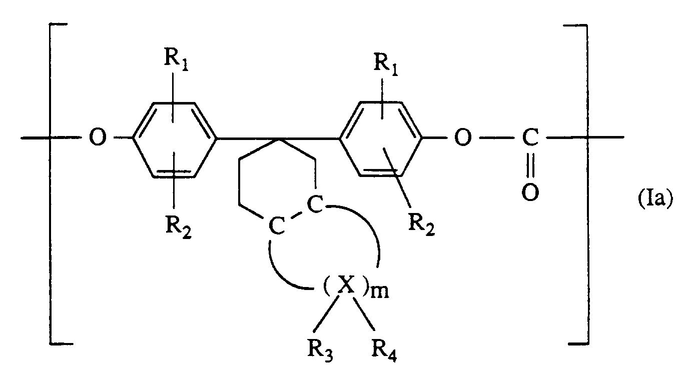
worin
X, R₁, R₂, R₃, R₄ und m die für die Formel (I) genannte Bedeutung haben,
in Mengen von 100 Mol-% bis 2 Mol-%, vorzugsweise in Mengen von 100 Mol-% bis 5 Mol-% und insbesondere in Mengen von 100 Mol-% bis 10 Mol-% und ganz besonders 100 Mol-% bis 20 Mol-%, bezogen jeweils auf die Gesamtmene von 100 Mol-% an difunktionellen Carbonatstruktureinheiten im Polycarbonat enthalten.

Die erfindungsgemäßen Polycarbonate enthalten somit jeweils zu 100 Mol-% komplementäre Mengen an anderen difunktionellen Carbonatstruktureinheiten, beispielsweise solche der Formel (XIa) 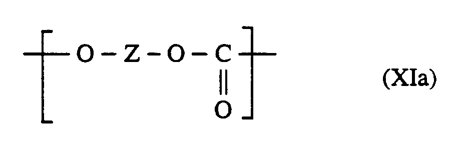
worin -Z- die für Formel (XI) gegebene Bedeutung hat, also in Mengen von 0 Mol-% (einschließlich) bis 98 Mol-% einschließlich, vorzugsweise von 0 Mol-% bis 95 Mol-% und insbesondere von 0 Mol-% bis 90 Mol-% und ganz besonders bevorzugt 0 Mol-% bis 80 Mol-%, bezogen jeweils auf die Gesamtmenge von 100 Mol-% an difunktionellen Carbonatstruktureinheiten im Polycarbonat. Polycarbonate auf Basis von cycloaliphatischen Bisphenolen sind grundsätzlich bekannt und z.B. in EP-0 164 476, DE-OS 33 595, DE-OS 2 063 052, FR 1 427 998, WP 8 000 348, BE 785 189 beschrieben. Sie haben häufig relativ hohe Einfriertemperaturen, aber andere, wichtige physikalische Eigenschatten wie UV- und Wärmealterungsstabilität sind unzureichend.

Es hat sich nun überraschenderweise gezeigt, daß wie bereits erwähnt durch den Einbau der erfindungsgemäßen Diphenole der Formel (I) neue Polycarbonate mit einem guten Eigenschaftsbild entstehen.

Durch die beliebige Kombination mit anderen Diphenolen, insbesondere mit denen der Formel (XI) lassen sich zudem die Polycarbonateigenschaften in günstiger Weise variieren.

Die Isolierung der nach dem erfindungsgemäßen Verfahren erhältlichen Polycarbonate geschieht in bekannter Weise, indem man die bei Phasengrenzflächenverfahren erhaltene organische Phase abtrennt, neutral und elektrolytfrei wäscht und dann beispielsweise über einen Eindampfextruder als Granulat isoliert oder die Polycarbonate aus organischer Lösung ausfällt und isoliert.

Den erfindungsgemäßen Polycarbonaten können noch vor oder nach ihrer Verarbeitung die für thermoplastische Polycarbonate üblichen Additive wie Stabilisatoren, Entformungsmittel, Pigmente, Flammschutzmitel, Antistatika, Füllstoffe und Verstärkungsstoffe in den üblichen Mengen zugesetzt werden.

Im einzelnen können beispielsweise Ruß, Kieselgut, Kaolin, Tone, CaF₂, CaCO₃, Aluminiumoxide, Glasfasern und anorganische Pigmente sowohl als Füllstoffe als auch als Nucleierungsmittel zugesetzt werden, sowie als Entformungsmittel beispielsweise Glycerinstearate, Pentaerythrittetrastearat und Trimethylolpropantristearat.

Die erfindungsgemäßen Polycarbonate können zu Formkörpern verarbeitet werden, indem man beispielsweise die in bekannter Weise isolierten Polycarbonate zu Granulat extrudiert und dieses Granulat gegebenenfalls nach Zusatz der obengenannten Additive durch Spritzguß zu verschiedenen Artikeln in bekannter Weise verarbeitet.

Die erfindungsgemäßen Polycarbonate sind als Formkörper überall dort einsetzbar, wo die bislang bekannten Polycarbonate eingesetzt wurden, also im Elektrosektor sowie im Bausektor für Abdeckungen und Verglasungen und im Haushaltssektor.

In den nachfolgenden Beispielen B.1 und B.2 wird die relative Viskosität gemessen an 0,5 gew.-%iger Lösung des Polycarbonats in CH₂Cl₂.

Die Einfriertemperatur oder Glastemperatur wird gemessen durch Differential Scanning Calorimetry (DSC).

### Beispiel B.1

18,90 g (0,05 Mol) des Diphenols (II), 12,0 g (0,3 Mol) NaOH und 182 ml Wasser werden in einer Inertgas-Atmosphäre unter Rühren gelöst. Dann fügt man eine Lösung von 0,106 g (2,25 Mol-%) Phenol in 136 ml Methylenchlorid zu. In die gut gerührte Lösung wurden bei pH 13 bis 14 und 21 bis 25°C 9,9 g (0,100 Mol) Phosgen eingeleitet. Dnach wird 0,2 ml N-Ethylpiperidin zugegeben und noch 45 Minuten gerührt. Die bisphenolatfreie wäßrige Phase wird abgetrennt, die organische Phase nach Ansäuern mit Phosphorsäure mit Wasser neutral gewaschen und vom Lösungsmittel befreit Das Polycarbonat zeigt eine relative Lösungsviskosität von 1,148.
Die Glastemperatur des Polymers wurde zu 242°C bestimmt (DSC).

### Beispiel B.2

10,92 g (0,03 Mol) des Diphenols (III), 0,09 g (0,000775 Mol) Na-Phenol at und 27,67 g 45 %ige Natronlauge (0,3 Mol) werden in 285 ml Wasser in einer Inertgasatmosphäre gelöst Dann fügt man 123 ml Methylenchlorid zu und leitet innerhalb von 9 Minuten 8 g (0,0809 Mol) Phosgen unter Rühren ein. Nach Zugabe von 0,742 g (0,000655 Mol) N-Ethylpiperidin wird noch 30 Minuten gerührt Die Temperatur während der Phosgenierung und Kondensation betrug 25°C; der pH-Wert nach der Reaktion betrug 13. Die bisphenolatfreie wäßrige Phase wird abgetrennt, die organische Phase nach Ansäuern mit Phosphorsäure mit Wasser neutral gewaschen und vom Lösungsmittel befreit Das Polycarbonat zeigt eine relative Lösungsviskosität von 1,260.
Die Glastemperatur des Polymers wurde zu 240°C bestimmt (DSC).

## Patentansprüche (Patentansprüche für folgende(n) Vertragsstaat(en): BE, DE, FR, GB, IT, NL)

1. Dihydroxydiphenylbicycloalkane der Formel 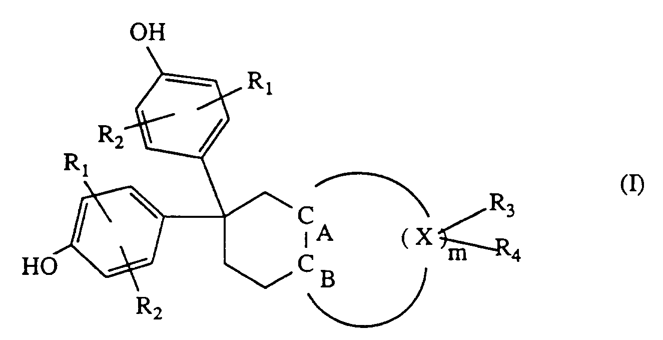 worin
R₁ und R₂ unabhängig voneinander Wasserstoff, Halogen, C₁-C₈-Alkyl, C₅-C₆-Cycloalkyl, C₆-C₁₀-Aryl oder C₇-C₁₂-Aralkyl sind,
m eine ganze Zahl von 3 bis 7
R₃ und R₄ für jedes X individuell wählbar, unabhängig voneinander Wasserstoff, lineares oder verzweigtes C₁-C₆-Alkyl, C₆-C₁₀-Aryl oder Aralkyl
und
X Kohlenstoff bedeuten,
mit der Maßgabe, daß an mindestens einem Atom X die Substituenten R₃ und R₄ nicht gleichzeitig Wasserstoff bedeuten.

2. Dihydroxydiphenylbicycloalkane der Formel (I) gemäß Anspruch 1, dadurch gekennzeichnet, daß in Formel (I) ein oder zwei der zu C_{A} und C_{B} α-ständigen X-Atome disubstituiert sind.

3. Dihydroxydiphenylbicycloalkane der Formel (I) gemäß Anspruch 1, dadurch gekennzeichnet, daß in Formel (I) m gleich 3 oder 4 ist.

4. Verfahren zur Herstellung der Dihydroxydiphenylbicycloalkane der Formel (I) des Anspruchs 1 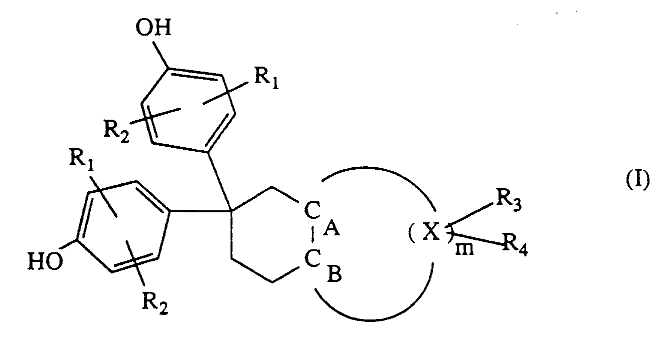 worin
X, R₁, R₂, R₃, R₄ und m die in Anspruch 1 genannte Bedeutung haben,
dadurch gekennzeichnet, daß man Phenole der Formel (VIII) 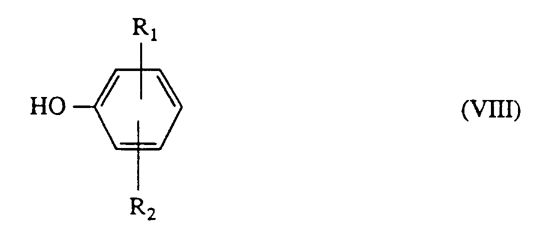 worin R₁ und R₂ die für Formel (I) angegebene Bedeutung haben,
mit Ketonen der Formel (IX) 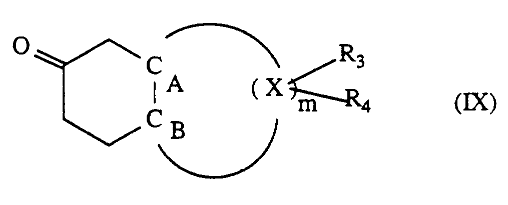 worin
X, m, R₃ und R₄ die für Formel (I) angegebene Bedeutung haben,
im Molverhältnis (VIII):(IX) zwischen 2:1 und 30:1 bei Temperaturen zwischen -30°C und 300°C und bei Drücken von 1 bis 20 bar in Anwesenheit saurer Katalysatoren und gegebenenfalls in Anwesenheit von Co-Katalysatoren und/oder Lösungsmitteln und/oder wasserentziehenden Mitteln umsetzt.

5. Verwendung der Diphenole der Formel (I) des Anspruchs 1 zur Herstellung von hochmolekularen, thermoplastischen, aromatischen Polycarbonaten.

6. Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, dadurch gekennzeichnet daß man als Diphenole solche der Formel (I) des Anspruchs 1 in Mengen von 100 Mol-% bis 2 Mol-%, bezogen jeweils auf die Gesamtmolmenge an eingesetzten Diphenolen, verwendet.

7. Hochmolekulare, thermoplastische, aromatische Polycarbonate mit M_{w} (Gewichtsmittelmolekulargewichten) von mindestens 9.000 erhältlich nach dem Verfahren des Anspruchs 6.

8. Hochmolekulare, thermoplastische, aromatische Polycarbonate mit M_{w} (Gewichtsmittelmolekulargewichten) von mindestens 9.000, die bifunktionelle Carbonatstruktureinheiten der Formel (Ia) 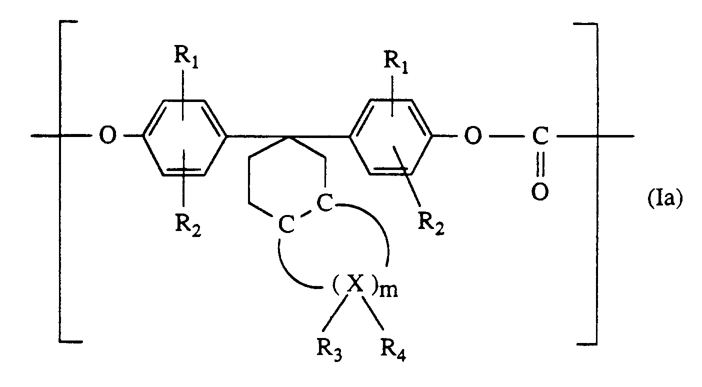 worin
X, R₁, R₂, R₃, R₄ und m die für die Formel (I) im Anspruch 1 genannte Bedeutung haben,
in Mengen von 100 Mol-% bis 2 Mol-%, bezogen jeweils auf die Gesamtmene von 100 Mol-% an difunktionellen Carbonatstruktureinheiten im Polycarbonat, enthalten.

## Patentansprüche (Patentansprüche für folgende(n) Vertragsstaat(en): ES)

1. Verfahren zur Herstellung von Dihydroxydiphenylbicycloalkanen der Formel (I) 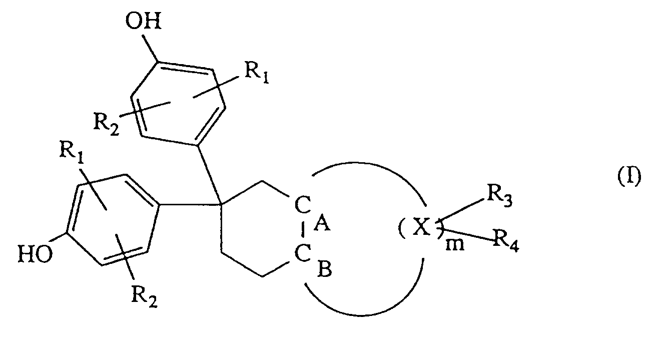 worin
R₁ und R₂ unabhängig voneinander Wasserstoff, Halogen, C₁-C₈-Alkyl, C₅-C₆-Cycloalkyl, C₆-C₁₀-Aryl oder C₇-C₁₂-Arakyl sind,
m eine ganze Zahl von 3 bis 7
R₃ und R₄ für jedes X individuell wählbar, unabhängig voneinander Wasserstoff, lineares oder verzweigtes C₁-C₆-Alkyl, C₆-C₁₀-Aryl oder Aralkyl
und
X Kohlenstoff bedeuten,
mit der Maßgabe, daß an mindestens einem Atom X die Substituenten R₃ und R₄ nicht gleichzeitig Wasserstoff bedeuten,
dadurch gekennzeichnet, daß man Phenole der Formel (VIII) 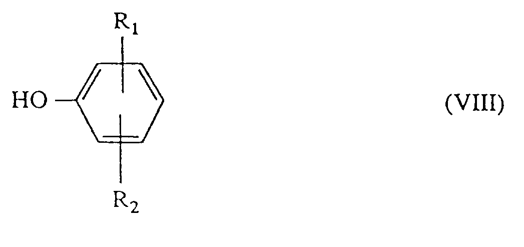 worin
R₁ und R₂ die für Formel (I) angegebene Bedeutung haben,
mit Ketonen der Formel (IX) 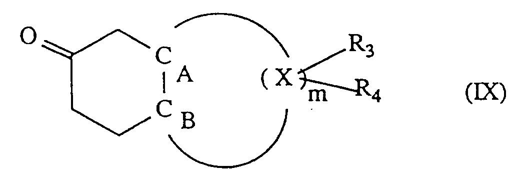 worin
X, m, R₃ und R₄ die für Formel (I) angegebene Bedeutung haben,
im Molverhältnis (VIII):(IX) zwischen 2:1 und 30:1 bei Temperaturen zwischen -30°C und 300°C und bei Drücken von 1 bis 20 bar in Anwesenheit saurer Katalysatoren und gegebenenfalls in Anwesenheit von Co-Katalysatoren und/oder Lösungsmitteln und/oder wasserentziehenden Mitteln umsetzt.

2. Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, dadurch gekennzeichnet, daß man als Diphenole solche der Formel (I) 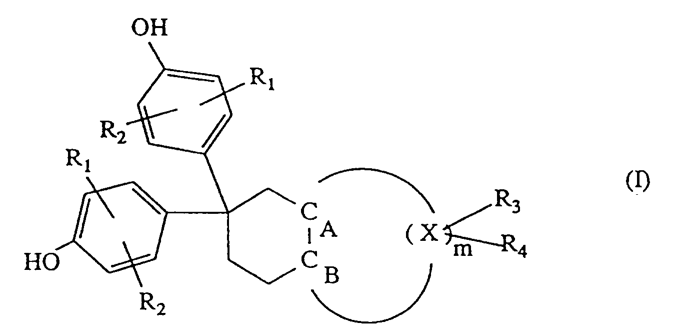 worin
R₁ und R₂ unabhängig voneinander Wasserstoff, Halogen, C₁-C₈-Alkyl, C₅-C₆-Cycloalkyl, C₆-C₁₀-Aryl oder C₇-C₁₂-Aralkyl sind,
m eine ganze Zahl von 3 bis 7
R₃ und R4 für jedes X individuell wählbar, unabhängig voneinander Wasserstoff, lineares oder verzweigtes C₁-C₆-Alkyl, C₆-C₁₀-Aryl oder Aralkyl
und
X Kohlenstoff bedeuten,
mit der Maßgabe, daß an mindestens einem Atom X die Substituenten R₃ und R₄ nicht gleichzeitig Wasserstoff bedeuten,
in Mengen Von 100 Mol-% bis 2 Mol-%, bezogen jeweils auf die Gesamtmolmenge an eingesetzten Diphenolen, verwendet.

## Claims (Claims for the following Contracting State(s): BE, DE, FR, GB, IT, NL)

1. Dihydroxydiphenyl bicycloalkanes corresponding to formula (I): 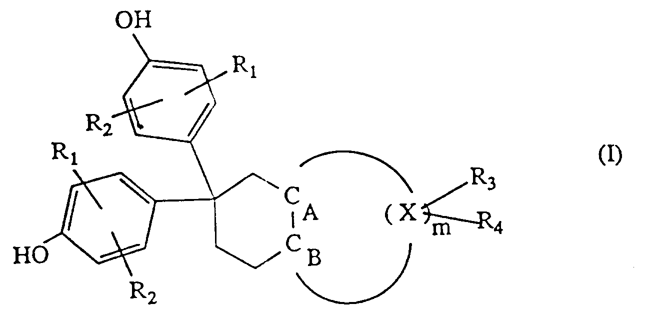 in which
R₁ and R₂ independently of one another represent hydrogen, halogen, C₁₋₈ alkyl, C₅₋₆ cycloalkyl, C₆₋₁₀ aryl or C₇₋₁₂ aralkyl,
m is an integer of from 4 to 7,
R₃ and R₄ may be individually selected for each X and, independently of one another represent, hydrogen, linear or branched C₁₋₆ alkyl, C₆₋₁₀ aryl or aralkyl,
and
X represents carbon,
with the proviso that, at at least one atom X, the substituents R₃ and R₄ are not both hydrogen.

2. Dihydroxydiphenyl bicycloalkanes corresponding to formula (I) in claim 1, characterized in that, in formula (I), one or two or the X atoms in the α-position to C-A and C-B are disubstituted.

3. Dihydroxydiphenyl bicycloalkanes corresponding to formula (I) in claim 1, characterized in that m in formula (I) is 4 or 5.

4. A process for the production of the dihydroxydiphenyl bicycloalkanes corresponding to formula (I) in claim 1: 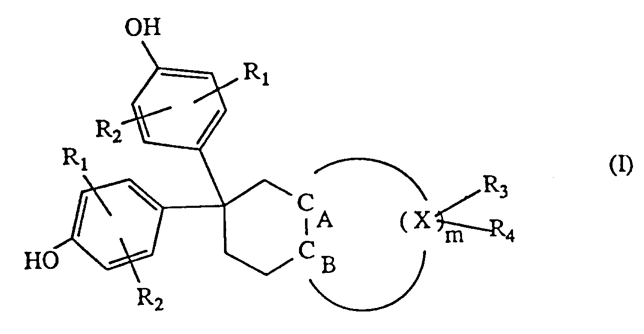 in which
X, R₁, R₂, R₃, R₄ and m are as defined in claim 1,
characterized in that phenols corresponding to formula (VIII): 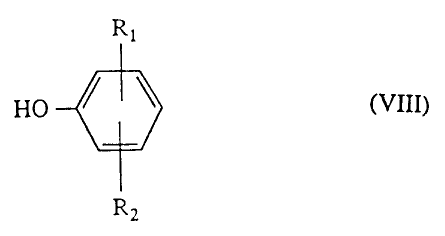 in which R₁ and R₂ are as defined for formula (I),
are reacted with ketones corresponding to formula (IX): 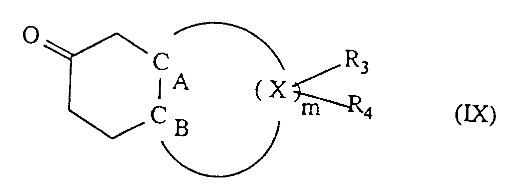 in which
X, m, R₃ and R₄ are as defined for formula (I),
in a molar ratio of (VIII):(IX) of from 2:1 to 30:1 at temperatures in the range from -30°C to 300°C and under pressures of from 1 to 20 bar in the presence of acidic catalysts and optionally in the presence of co-catalysts and/or solvents and/or dehydrating agents.

5. The use of the diphenols corresponding to formula (I) in claim 1 for the production of high molecular weight, thermoplastic aromatic polycarbonates.

6. A process for the production of high molecular weight, thermoplastic aromatic polycarbonates from diphenols, optionally chain terminators and optionally branching agents by the known methods for the production of polycarbonates, characterized in that diphenols corresponding to formula (I) in claim 1 are used as the diphenols in quantities of 100 mol-% to 2 mol-%, based on the total mols of diphenols used.

7. High molecular weight, thermoplastic aromatic polycarbonates having M_{w} values (weight average molecular weights) of at least 9,000 obtainable by the process claimed in claim 6.

8. High molecular weight, thermoplastic aromatic polycarbonates having M_{w} values (weight average molecular weights) of at least 9,000 which contain bifunctional carbonate structural units corresponding to formula (Ia): 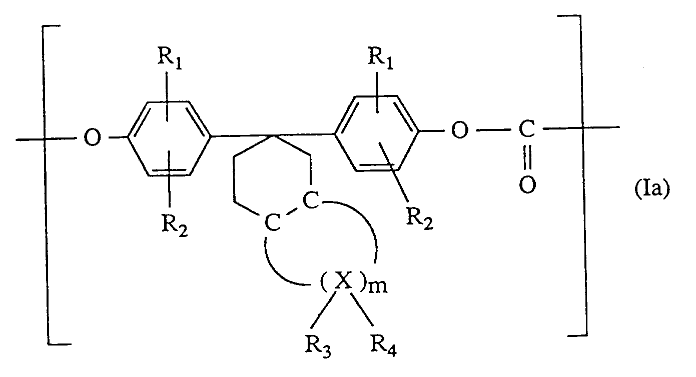 in which
X, R₁, R₂, R₃, R₄ and m are as defined for formula (I) in claim 1,
in quantities of 100 mol-% to 2 mol-%_{,} based on the total quantity of 100 mol-% of difunctional carbonate structural units in the polycarbonate.

## Claims (Claims for the following Contracting State(s): ES)

1. A process for the production of dihydroxydiphenyl bicycloalkanes corresponding to formula (I): 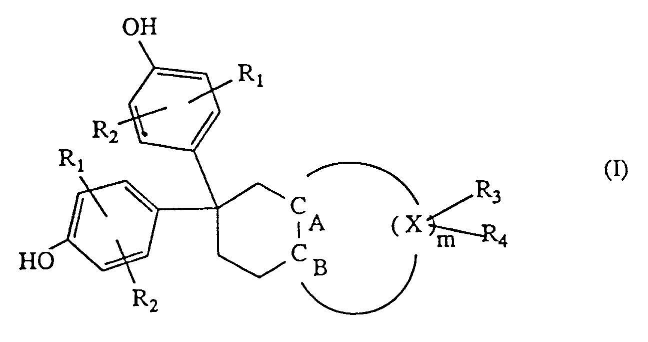 in which
R₁ and R₂ independently of one another represent hydrogen, halogen, C₁₋₈ alkyl, C₅₋₆ cycloalkyl, C₆₋₁₀ aryl or C₇₋₁₂ aralkyl,
m is an integer of from 4 to 7,
R₃ and R₄ may be individually selected for each X and, independently of one another represent, hydrogen, linear or branched C₁₋₆ alkyl, C₆₋₁₀ aryl or aralkyl,
and
X represents carbon,
with the proviso that, at at least one atom X, the substituents R₃ and R₄ are not both hydrogen,
characterized in that phenols corresponding to formula (VIII): 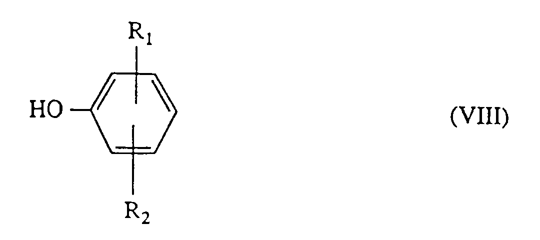 in which R₁ and R₂ are as defined for formula (I),
are reacted with ketones corresponding to formula (IX): 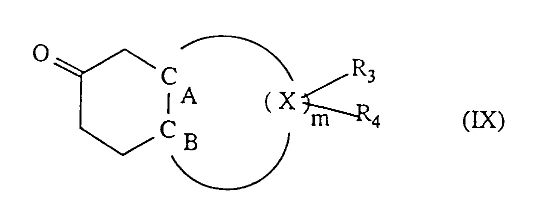 in which
X, m, R₃ and R₄ are as defined for formula (I),
in a molar ratio of (VIII):(IX) of from 2:1 to 30:1 at temperatures in the range from -30°C to 300°C and under pressures of from 1 to 20 bar in the presence of acidic catalysts and optionally in the presence of co-catalysts and/or solvents and/or dehydrating agents.

2. A process for the production of high molecular weight, thermoplastic aromatic polycarbonates from diphenols, optionally chain terminators and optionally branching agents by the known methods for the production of polycarbonates, characterized in that diphenols corresponding to formula (I): 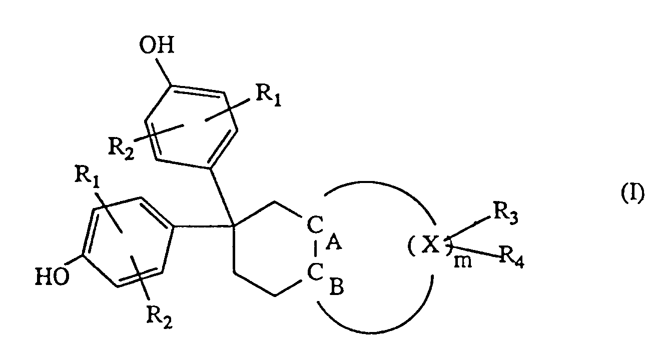 in which
R₁ and R₂ independently of one another represent hydrogen, halogen, C₁₋₈ alkyl, C₅₋₆ cycloalkyl, C₆₋₁₀ aryl or C₇₋₁₂ aralkyl,
m is an integer of from 4 to 7,
R₃ and R₄ may be individually selected for each X and, independently of one another represent, hydrogen, linear or branched C₁₋₆ alkyl, C₆₋₁₀ aryl or aralkyl,
and
X represents carbon,
with the proviso that, at at least one atom X, the substituents R₃ and R₄ are not both hydrogen,
are used as the diphenols in quantities of 100 mol-% to 2 mol-%, based on the total mols of diphenols used.

## Revendications (Revendications pour l'(les) Etat(s) contractant(s) suivant(s): BE, DE, FR, GB, IT, NL)

1. Dihydroxydiphénylbicycloalcanes de formule : 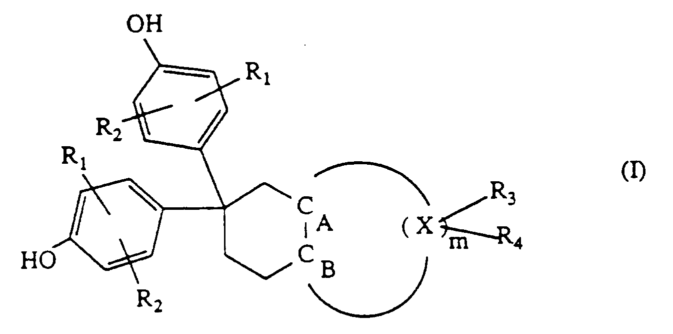 dans laquelle
R₁ et R₂ représentent chacun, indépendamment l'un de l'autre, un atome d'hydrogène, un atome d'halogène, un groupe alkyle en C₁ à C₈, cycloalkyle en C₅ ou C₆, aryle en C₆ à C₁₀ ou aralkyle en C₇ à C₁₂;
m est un nombre entier valant 3 à 7;
R₃ et R₄ peuvent, pour chaque X, être choisis individuellement, indépendamment l'un de l'autre, parmi un atome d'hydrogène, un groupe alkyle linéaire ou ramifié en C₁ à C₆, aryle en C₆ à C₁₀ ou aralkyle, et
X représente un atome de carbone,
à la condition que, sur au moins un atome X, les substituants R₃ et R₄ ne représentent pas simultanément chacun un atome d'hydrogène.

2. Dihydroxydiphénylbicycloalcanes de formule (I) selon la revendication 1, caractérisés en ce que, dans la formule (I), un ou deux des atomes X situés en α par rapport à C_{A} et C_{B} est ou sont disubstitué(s).

3. Dihydroxydiphénylbicycloalcanes de formule (I), selon la revendication 1, caractérisés en ce que, dans la formule (I), m vaut 3 ou 4.

4. Procédé de préparation des dihydroxydiphénylbicycloalcanes (I) de la revendication 1 : 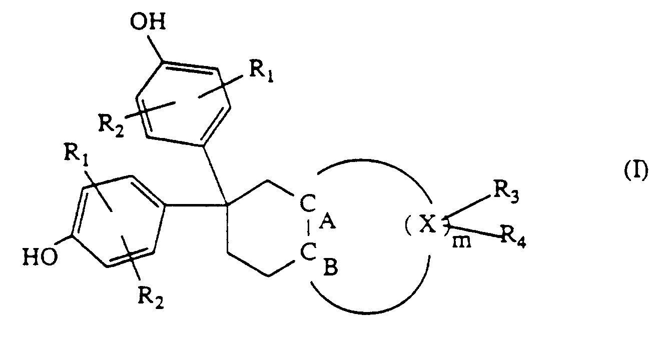 dans laquelle
X, R₁, R₂, R₃, R₄ et m ont le sens indiqué à la revendication 1,
procédé caractérisé en ce qu'on fait réagir des phénols de formule (VIII): 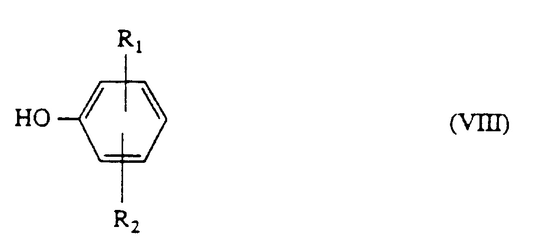 dans laquelle
R₁ et R₂ ont le sens indiqué à propos de la formule (I),
avec des cétones de formule (IX): 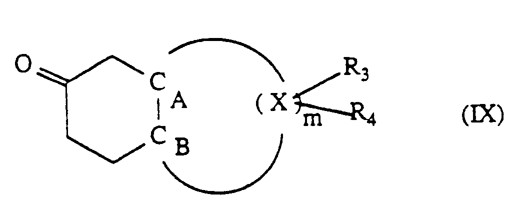 dans laquelle
X, m, R₃ et R₄ ont le sens indiqué pour la formule (I),
selon un rapport molaire (VIII):(IX) compris entre 2:1 et 30:1, à des températures comprises entre -30°C et +300°C et à des pressions de 1 à 20 bars, en présence de catalyseurs acides et éventuellement en présence de catalyseurs à base de Co et/ou de solvants et/ou d'agents d'enlèvement de l'eau.

5. Utilisation des diphénols de formule (I) de la revendication 1 pour produire des polycarbonates aromatiques thermoplastiques à poids moléculaire élevé.

6. Procédé de préparation de polycarbonates aromatiques thermoplastiques, à poids moléculaire élevé, que l'on obtient à partir de diphénols, éventuellement d'agents de rupture des chaînes et éventuellement d'agents de ramification, selon les procédés connus pour la préparation des polycarbonates, procédé caractérisé en ce qu'on utilise comme diphénols ceux de formule (I) de la revendication 1, en des quantités de 100 mol % à 2 mol %, chaque fois par rapport à la quantité molaire totale des diphénols que l'on utilise.

7. Polycarbonates aromatiques thermoplastiques à poids moléculaire élevé, présentant un M̅ₚ̅ (poids moléculaire moyen en poids) valant au moins 9 000, que l'on peut obtenir selon le procédé de la revendication 6.

8. Polycarbonates aromatiques thermoplastiques à poids moléculaire élevé, ayant un M̅ₚ̅ (poids moléculaire moyen en poids) valant au moins 9 000, qui contiennent des motifs structurels carbonates bifonctionnels de formule (Ia): 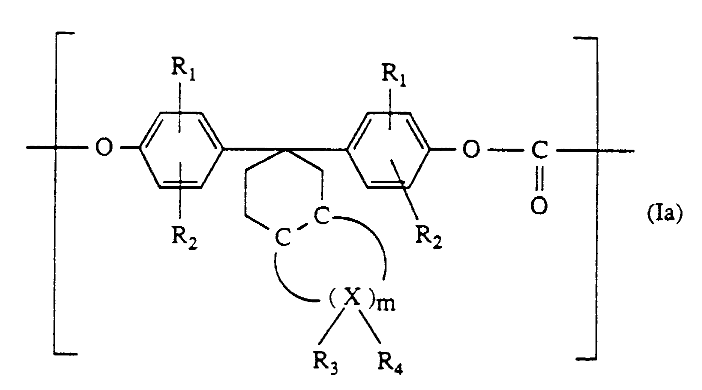 dans laquelle
X, R₁, R₂, R₃, R₄ et m ont le sens indiqué pour la formule (I) de la revendication 1,
en des quantités de 100 mol % à 2 mol %, chaque fois par rapport à la quantité totale de 100 mol % en des motifs structurels carbonates difonctionnels dans le polycarbonate.

## Revendications (Revendications pour l'(les) Etat(s) contractant(s) suivant(s): ES)

1. Procédé de production de dihydroxydiphénylbicycloalcanes de formule(I): 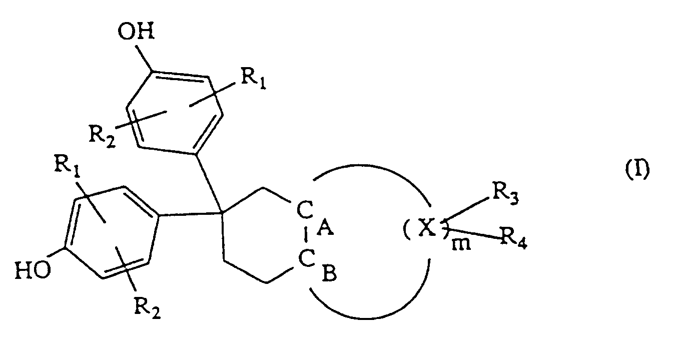 dans laquelle
R₁ et R₂ représentent chacun, indépendamment l'un de l'autre, un atome d'hydrogène, un atome d'halogène, un groupe alkyle en C₁ à C₈, cycloalkyle en C₅ ou C₆, aryle en C₆ à C₁₀ ou aralkyle en C₇ à C₁₂,
m est un nombre entier valant 3 à 7,
R₃ et R₄ peuvent être choisis individuellement, pour chaque X, indépendamment l'un de l'autre, parmi un atome d'hydrogène, un groupe alkyle linéaire ou ramifié en C₁ à C₆, aryle en C₆ à C₁₀ ou aralkyle, et
X représente un atome de carbone,
à la condition que, sur au moins un atome X, les substituants R₃ et R₄ ne signifient pas simultanément chacun un atome d'hydrogène,
procédé caractérisé en ce qu'on fait réagir des phénols de formule (VIII): 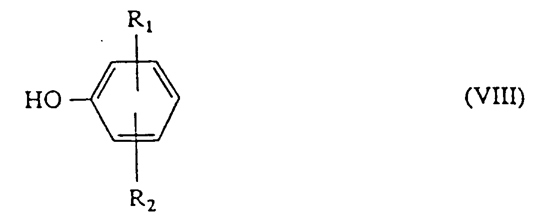 dans laquelle
R₁ et R₂ ont le sens indiqué pour la formule (I),
avec des cétones de formule (IX): 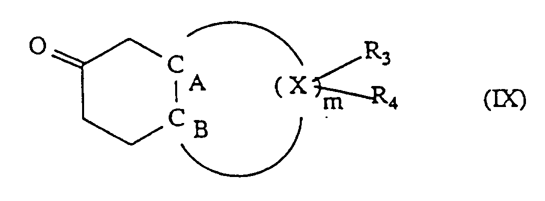 dans laquelle
X, m, R₃ et R₄ ont le sens indiqué pour la formule (I),
selon un rapport molaire (VIII):(IX) compris entre 2:1 et 30:1 à des températures comprises entre -30°C et +300°C et sous des pressions de 1 à 20 bars, en présence de catalyseurs acides et éventuellement en présence de catalyseurs à base de Co et/ou de solvants et/ou d'agents enlevant l'eau.

2. Procédé de préparation de polycarbonates aromatiques thermoplastiques à poids moléculaire élevé, à partir de diphénols, éventuellement d'agents de rupture des chaînes et éventuellement d'agents de ramification, selon les méthodes connues pour la préparation des polycarbonates, procédé caractérisé en ce qu'on utilise comme diphénols ceux de formule (I): 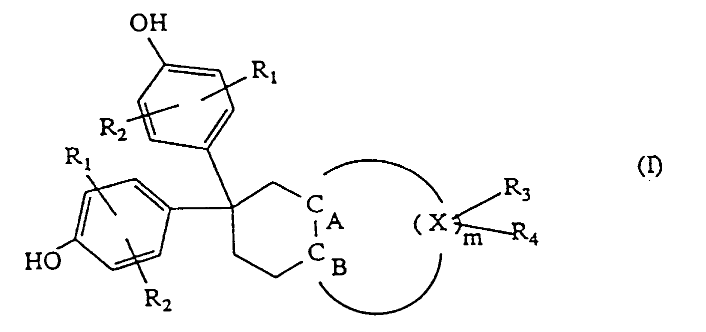 dans laquelle
R₁ et R₂ représentent chacun, indépendamment l'un de l'autre, un atome d'hydrogène, un atome d'halogène, un groupe alkyle en C₁ à C₈, cycloalkyle en C₅ ou C₆, aryle en C₆ à C₁₀ ou aralkyle en C₇ à C₁₂,
m est un nombre entier valant 3 à 7,
les symboles R₃ et R₄ peuvent, pour chaque X, être choisis individuellement, indépendamment l'un de l'autre, parmi un atome d'hydrogène, un groupe alkyle linéaire ou ramifié en C₁ à C₆, un groupe aryle en C₆ à C₁₀ ou aralkyle, et X représente un atome de carbone,
à la condition que, sur au moins un atome X, les substituants R₃ et R₄ ne représentent pas simultanément chacun un atome d'hydrogène,
en des quantités de 100 mol % à 2 mol %, chaque fois par rapport à la quantité molaire totale des diphénols que l'on utilise.
